# EUROPEAN PATENT APPLICATION

(11) **EP 4 029 849 A1**
(43) Date of publication of application: **20.07.2022**
(21) Application number: 20861266.3
(22) Date of filing: 09.04.2020
(51) Int. Cl.: C07C 37/045, C07C 39/24

(54) **METHOD FOR PREPARING M-TRIFLUOROMETHYLPHENOL**

(30) Priority: 04.09.2019 CN 201910831538
(71) Applicant: Zhejiang Weihua New Material Co., Ltd., Shaoxing, Zhejiang 312369 (CN)
(72) Inventor: PAN, Qiangbiao, Zhejiang 312369 (CN); CHEN, Jinghua, Zhejiang 312369 (CN); ZHANG, Zengxing, Zhejiang 312369 (CN); LI, Junqi, Zhejiang 312369 (CN)
(74) Representative: dompatent von Kreisler Selting Werner - Partnerschaft von Patent- und Rechtsanwälten mbB
(86) International application number: PCT/CN2020/083955
(87) International publication number: WO 2021/042721

(57) **Abstract**

Disclosed is a method for preparing m-trifluoromethylphenol. The present invention provides a method for preparing m-trifluoromethylphenol, which comprises the following steps: step (1): in a continuous flow reactor, subjecting an aqueous sulfate solution and an aqueous sodium nitrite solution to a diazotization reaction to obtain a diazonium salt solution, wherein the aqueous sulfate solution is a mixture of a sulfuric acid aqueous solution and m-trifluoromethyl aniline; and step (2): in the continuous flow reactor, subjecting the diazonium salt solution obtained in step (1) to a hydrolysis reaction in the presence of an aromatic solvent to obtain the m-trifluoromethylphenol, wherein the temperature of the hydrolysis reaction is 101°C-200°C. The continuous preparation method can increase the reaction concentration, greatly shorten the reaction time, improve the production efficiency and reduce safety risks; and the method can achieve a yield of 95% or more and a purity of 99.5% or more, is easy and simple to operate, and has the advantages of being safer, more environmentally friendly, of a lower cost, etc.

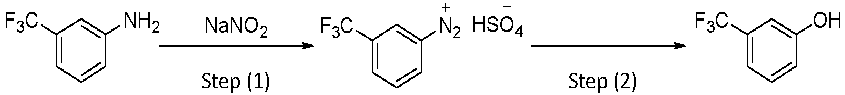

## Description

The present application claims the benefit of Chinese patent application 201910831538X filed on September 04, 2019. The contents of the Chinese patent application are incorporated herein by reference in their entireties.

### Technical Field

The invention relates to a preparation method of *m*-trifluoromethylphenol.

### Background

Due to the special physical and chemical properties of fluorinated compounds, their applications in pesticides and pharmaceuticals are receiving more and more attention. For example, *m*-trifluoromethylphenol is one of the main derivatives of m-trifluoromethylaniline, which is not only used to synthesize herbicides and pesticides, but also used to synthesize some antibiotics and anti-cancer drugs as an important intermediate.

Considering the impacts of impurities on the subsequent purification during the production of pesticides and pharmaceuticals, the marketing target of m-trifluoromethylphenol generally requires more than 99.5%.

Literature provides 5 methods for the synthesis of *m*-trifluoromethylphenol: hydrogen peroxide oxidation, trifluoromethyl alkylation, electrolysis, ether hydrolysis, and diazotization hydrolysis.

Among them, diazotization hydrolysis is the most conventional method for the synthesis of phenols, with easily available raw materials, low cost and applicability for large-scale production, which is currently the most widely used method in industry. However, in the intermittent tank reactor, the diazotization reaction is violent and not easy to control, and the temperature required for diazotization is low; the diazotization salt is explosive and unsafe for large-scale production, which is a dangerous chemical process; and the hydrolysis of diazotization salt is rapidly exothermic and outgassing (requiring high temperature), with poor safety and high environmental pollution; and the hydrolysis is accompanied with a side coupling reaction of phenol with diazonium salt, resulting a yield generally about 80% and a purity lower than 99%.

The invention patent CN106905096A reports a method of continuous flow synthesis of 3-(trifluoromethyl)phenol in two static mixers, tubular reactors and oil-water separators series-connected in sequence, comprising the following steps: (1) pumping an acid solution and 3-(trifluoromethyl)aniline into the static mixer at 0°C-50°C; (2) discharging the mixture of the acid solution and 3-(trifluoromethyl)aniline from a static mixer A into a static mixer B connected with the static mixer A, pumping sodium nitrite solution into the static mixer B at 0°C-50°C, to generate diazonium salt solution; (3) discharging the diazonium salt solution from the static mixer B into a tube reactor connected with the static mixer B with a reaction temperature of 80°C-100°C, and then into an oil-water separator connected with the tube reactor, separating the aqueous phase to obtain 3-(trifluoromethyl)phenol.

The literature "Jinsha, Chen, Xiaohua, Du. Continuous Synthesis of m-Trifluoromethylphenol in Microchannel Reactor [J]. AGROCHEMICALS, 2017, 56(5): 320-323" reports microchannel continuous synthesis of *m*-trifluoromethylphenol, comprising (1) dissolving 25% sulfuric acid solution and *m*-trifluoromethylaniline at 60°C in a four-neck flask; (2) lowering the temperature to 12.5°C, reacting the obtained suspension of the amine salt with 30% sodium nitrite solution by microchannel continuous synthesis to prepare diazonium salt; (3) then hydrolyzing at 95°C in a threeneck flask. The purity is 99.1% and the yield is 92.5%.

The literature also provides a method for the preparation of m-trifluoromethylphenol by microchannel continuous synthesis, comprising (1) mixing 25% sulfuric acid solution with *m*-trifluoromethylaniline in a four-neck flask, cooling to 3°C, adding 30% sodium nitrite solution, heating to 20°C for the diazonium salt reaction, filtering, and obtaining the diazonium salt solution; (2) hydrolyzing the aqueous diazonium salt solution in a microchannel reactor at 88°C. The purity is 99.2% and the yield is 93.6%.

The above-mentioned synthesis of *m*-trifluoromethylphenol by diazotization hydrolysis using the microchannel continuous method, by taking advantage of the low safety risk and high heat transfer efficiency of microreactors, improves the selectivity, rate and yield of the reaction and can save solvent, adapt to reactions at a higher temperature and have shorter reaction time to a certain extent, compared with conventional reactors. However, the method still has some shortcomings:
(1) In the salt formation reaction, the existing processes are carried out by feeding the raw materials in a form of suspension or slurry, and the presence of solid particles in the raw materials can easily lead to clogging of the microchannel reactor, which poses a very high risk.
(2) In the diazotization reaction, the existing continuous diazotization processes are mostly carried out at a lower temperature, and the concentration of the raw materials in the reaction solution is dilute in order to avoid clogging of the reactor due to the precipitation of the salt generated after the reaction, which are difficult to meet the flux demand of industrial scale-up.
(3) In the hydrolysis reaction, the hydrolysis temperature in the existing continuous flow processes is generally not higher than 100°C, trifluoromethylbenzene diazonium salt undergoes incomplete hydrolysis at 100°C and below, by-products are more likely to generate, so the highest reaction yield is 93.6%, the product purity is 99.2%, and it is difficult to steadily obtain products with purity of 99.5% or more; thus, the method cannot meet the needs of pharmaceutical intermediates and other high-end market.

With the increasing requirements for the purity of such products, as well as the increasingly strict national supervision of chemical production safety and environmental protection, how to further improve the purity and yield, while strengthening the level of control of hazardous chemical processes, reducing safety risks and reducing the emission of three wastes, is an urgent problem to be solved for manufacturers.

### Content of the present invention

The technical problem to be solved by the present invention is to overcome the defects of the existing diazotization hydrolysis methods for the preparation of phenolic compounds, such as high safety risk, excessive three wastes, long reaction time, low purity, *etc.* Instead, the invention discloses a method for the continuous preparation of phenolic compounds using a continuous flow reactor, and the continuous preparation method can allow higher concentration, greatly shorten the reaction time, improve the production efficiency, reduce the safety risk, and has the advantages of high yield and purity, easy operation, increased safety, better environmental protection, lower cost, *etc.*

The present invention solves the above technical problems through the following technical solution.

The present invention provides a preparation method of m-trifluoromethylphenol, comprising the following steps:
step (1): in a continuous flow reactor, carrying out a diazotization reaction of sulfate aqueous solution and sodium nitrite aqueous solution to obtain diazonium salt solution; said sulfate aqueous solution is a mixture of sulfuric acid aqueous solution and *m*-trifluoromethylaniline;
in the sulfate aqueous solution, the molar ratio of sulfuric acid in the sulfuric acid aqueous solution to the *m*-trifluoromethylaniline is (3-7): 1;
the molar ratio of sodium nitrite in the sodium nitrite aqueous solution to the *m*-trifluoromethylaniline is (1.00-1.50): 1;
step (2): in a continuous flow reactor, carrying out a hydrolysis reaction of the diazonium salt solution obtained in the step (1) in the presence of an aromatic hydrocarbon solvent to obtain the *m*-trifluoromethylphenol; the temperature of the hydrolysis reaction is 101°C-200°C;

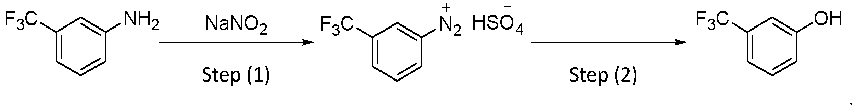

The sulfate aqueous solution can be a homogeneous system formed by the sulfuric acid aqueous solution and the *m*-trifluoromethyl aniline.

In the sulfate aqueous solution, the molar ratio of the sulfuric acid to the m-trifluoromethylaniline can be (3-6):1 *(e.g.,* 4:1, 5:1 or 6:1); preferably (3-4):1.

The mass percentage concentration of the sulfuric acid aqueous solution can be 20%-40%, preferably 21%-25%.

In the sulfate aqueous solution being a mixture of sulfuric acid aqueous solution and *m*-trifluoromethylaniline, the mixture can be a salt formed by m-trifluoromethylaniline and sulfuric acid, or, *m*-trifluoromethylaniline sulfate, and sulfuric acid or trifluoromethylaniline.

The mass percentage concentration of the sodium nitrite aqueous solution can be 20%-35%, preferably 25%-30%.

The molar ratio of the sodium nitrite to the *m*-trifluoromethylaniline in the continuous flow reactor can be 1:04:1, 1:05:1, 1.06:1, 1.07:1, 1.09:1, 1.10:1, 1.11:1, 1.12:1, 1.13:1, 1.14:1, 1.16:1, 1.18:1, 1.20:1, 1.22: 1, 1.24:1, 1.26:1, 1.28:1, 1.30:1, 1.32:1, 1.34:1, 1.36:1, 1.38:1, 1.40:1, 1.42:1, 1.44:1, 1.46:1, 1.48:1, or 1.5:1; preferably (1.04-1.15):1; more preferably (1.04-1.10): 1.

The temperature of the diazotization reaction can be 0°C-100°C *(e.g.,* 0°C, 5°C, 10°C, 15°C, 20°C, 25°C, 30°C, 35°C, 40°C, 45°C, 50°C, 55°C, 60°C, 65°C, 70°C, 75°C, 80°C, 85°C, 90°C, 95°C, or 100°C); preferably 50°C-70°C.

It should be understood by those skilled in the art that in this type of reaction, there is a certain correspondence between residence time and reaction temperature; the higher the reaction temperature, the shorter the corresponding residence time; and the lower the reaction temperature, the longer the corresponding residence; ensuring that the reaction is as complete as possible while the impurities are as fewer as possible. In the present invention, the residence time for the diazotization reaction of the sulfate aqueous solution and the sodium nitrite aqueous solution in the continuous flow reactor is such that the *m*-trifluoromethylaniline disappears or ceases to react, for example, the residence time can be 5.3 s-7.5 s *(e.g.,* 1 s, 2 s, 3 s, 4 s, 5 s, 6 s, 7 s).

Those skilled in the art will appreciate that the sulfate aqueous solution and the sodium nitrite aqueous solution are preferably pumped simultaneously into the continuous flow reactor.

The flow rate ratio of the sulfate aqueous solution to the sodium nitrite aqueous solution can be 4:1 to 12:1 *(e.g.,* 4.3, 5.3, 5.4, 5.7, 5.8, 5.9, 6.0, 6.1, 6.5, 6.9, 7.6, 7.8, 7.9, 8.5, 9.0, 9.5, 10.2, 10.5, 10.7, 11.0, 11.6, or 11.7).

The aromatic hydrocarbon solvent can be an aromatic hydrocarbon solvent conventional in the art, such as toluene and/or xylene (e.g., mixed xylene).

The temperature of the hydrolysis reaction can be 101°C, 105°C, 110°C, 115°C, 120°C, 125°C, 130°C, 135°C, 140°C, 145°C, 150°C, 155°C, 160°C, 165°C, 170°C, 175°C, 180°C, 185°C, 190°C, 195°C, or 200°C, preferably 120°C-150°C.

The residence time of the hydrolysis reaction is the time required to complete the hydrolysis reaction in the continuous flow reactor on the basis that the diazonium salt disappears or ceases to react; the residence time of the hydrolysis reaction can be 1 min-50 min *(e.g.,* 1 min, 2 min, 3 min, 4 min, 5 min, 6 min, 8 min, 10 min, 15 min, 25 min, 30 min, 35 min, 40 min, or 50 min), preferably 1 min-5 min.

The volume ratio of the diazonium salt solution to the aromatic hydrocarbon solvent can be (0.1-100):1 *(e.g.,* 0.1:1, 4:1, 16.6:1 or 83.6:1); preferably 4:1 to 20:1.

In some embodiments, the aromatic hydrocarbon solvent can be preheated in a heat exchanger before mixing with the diazonium salt solution, and then the hydrolysis reaction is carried out in the continuous flow reactor; for example: preheating the mixture to 90-130°C; the residence time of the aromatic hydrocarbon solvent preheated in the heat exchanger can be 0.5 to 300 s.

The preparation method can further comprise a post-treatment step, wherein, after the hydrolysis reaction is completed, the reaction system is subjected to oil-water separation, for example, in a conventional oil-water separator in the art, and then the oil phase is desolventized and distilled to obtain the *m*-trifluoromethylphenol.

In the preparation method, the step (1) can further comprise the following step: in a continuous flow reactor, carrying out a salt-forming reaction of sulfuric acid aqueous solution and *m*-trifluoromethyl aniline to obtain the sulfate aqueous solution.

The temperature of the salt-forming reaction can be 5°C-150°C *(e.g.,* 5°C, 10°C, 15°C, 20°C, 25°C, 30°C, 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C 130°C, 135°C, 140°C, 145°C, or 150°C); preferably 80°C-100°C (e.g., 85°C-95°C).

The flow rate ratio of the sulfuric acid aqueous solution to the m-trifluoromethylaniline can be 8:1 to 20:1 *(e.g.,* 8.0, 8.1, 8.2, 8.3, 8.4, 8.7, 10.9, 11.1, 11.2, 13.7, 13.8, 15.9, 16.4, 16.5, 19.1, 19.3, 19.4 or 19.7).

In some embodiments, the sulfuric acid aqueous solution and the m-trifluoromethylaniline are mixed and preheated in a static mixer separately, and then the salt-forming reaction is carried out; for example, preheated to 85°C to 95°C.

It can be understood by those skilled in the art that the continuous flow reactor may independently be a continuous flow reactor conventional in the art such as a static mixer, a microchannel reactor or a tubular reactor or a combination thereof; a microchannel reactor is preferred in the step (1), and a tubular reactor is preferred in the step (2).

When the diazonium salt solution and the aromatic hydrocarbon solvent are mixed and preheated in a continuous flow reactor, the continuous flow reactor can be a microchannel reactor.

It can be understood by those skilled in the art that according to the production scale, one or more groups of the continuous flow reactors can be connected in parallel to form a continuous flow reaction system, to prepare the *m*-trifluoromethylphenol.

Without violating the common sense in the art, the above-mentioned preferred conditions can be arbitrarily combined to obtain the preferred examples of the present invention.

The reagents and raw materials used in the invention are commercially available.

The positive and progressive effects of the invention are as follows: the method for synthesizing m-trifluoromethyl phenol in continuous flow provided by the invention: 1) the method increases the concentration of salt formation and diazotization reaction, increases the reaction rate and reduces the output of waste water; 2) the method raises the temperature of salt formation and diazotization reaction, eliminates the risk of blockage of the reactor by the solid salt, and greatly improves the safety of the reactor; (3) this method raises the temperature of hydrolysis reaction, further reduces the by-products caused by incomplete hydrolysis, and improves the yield (over 95%) and purity (over 99.5%); moreover, *m*-trifluoromethyl phenol with purity over 99.5% can be obtained after easy purification, which can directly meet the market demand and save the energy consumption and cost caused by purification.

### Brief description of the drawings

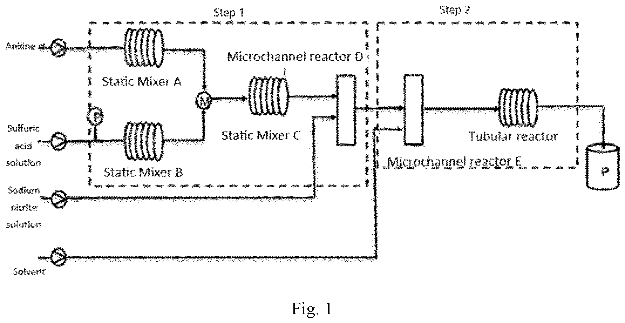

Fig. 1 is a schematic diagram of the process flow of examples 1-37.

### Detailed description of the preferred embodiment

The present invention is further described below by way of embodiments but is not thus limited to the scope of the described embodiments. The experimental methods in the examples below of which the specific conditions are not indicated are selected according to the conventional methods and conditions, or according to the commodity instructions.

The purity of the product in this example was determined by high performance liquid chromatography (HPLC). The HPLC conditions are as follows:
1. Column: ZORBAX Eclopse XDB-C18 (4.6 mm^{*}250 mm-5 um); 2. column temperature: 25 °C; 3. mobile phase: water : methanol = 30:70 (v/v); 4. flow rate: 1.0 ml/min; 5. injection volume: 1 µl; 6. detection wavelength: 254 nm; 7. detection time: 13 min. Xylene is mixed xylene.

### Embodiments 1-37

Sulfuric acid aqueous solution and aniline compound (1 equivalent) were pumped into static mixers A and B at corresponding flow rates, respectively, and then mixed in static mixer C at a certain temperature to carry out salt-forming reaction, and a uniform mixture was obtained. The resulting mixture outflowed from the static mixer C (where the flow rate was the sum of A and B), and flowed into the microchannel reactor D together with nitrite aqueous solution at corresponding flow rates, respectively, and the diazotization reaction occurred to obtain diazonium salt solution. The obtained diazonium salt solution outflowed from the microchannel reactor D (the flow rate here was the sum of A, B and C), and then flowed into the microchannel reactor E together with the organic solvent at the corresponding flow rates to be mixed, and then outflowed from the microchannel reactor E after uniform mixing, and flowed into the tubular reactor F at a certain temperature for hydrolysis reaction. After the reaction was completely, the reaction mixture outflowed from the tubular reactor F and flowed into the oil-water separator for oil-water separation, and the oil phase was desolventized and distilled to obtain phenol compounds. The reaction parameters and results are as follows:

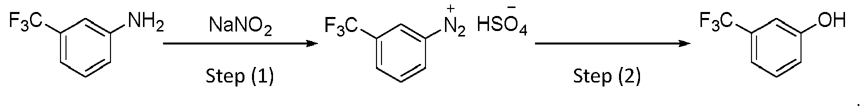

| Embo diment | Salt forming temperatu re | Diazotizat ion temperat ure | Time of step 1 | Hydrolysis temperatu re | Time of step 2 | Sodium nitrite equivalent s | Sulfuri c acid equival ents | Sulfuric acid solution concentr ation | Aniline compound * | Sulphuric acid solution^{∗} | Sodium nitrite solution^{∗} | Organic solvent | Organic solvent | Yield of produ ct | Purity of produ ct |
|---|---|---|---|---|---|---|---|---|---|---|---|---|---|---|---|
| | °C | °C | s | °C | min | eq. | eq. | % | ml/min | ml/min | ml/min | | **ml/min** | % | % |
| 1 | 5 | 0 | 1 | 101 | 4 | 1.04 | 3 | 20 | 5.17 | 42 | 8 | Toluene | 13.79 | 96 | 99.5 |
| 2 | 10 | 0 | 1 | 101 | 4 | 1.05 | 3 | 21 | 6.9 | 58 | 10 | Toluene | 18.73 | 95 | 99.6 |
| 3 | 15 | 5 | 3 | 105 | 8 | 1.07 | 4 | 24 | 9.2 | 100 | 14 | Toluene | 30.8 | 97 | 99.6 |
| 4 | 20 | 10 | 3 | 110 | 8 | 1.07 | 5 | 24 | 12.3 | 168 | 20 | Toluene | 50.08 | 96 | 99.8 |
| 5 | 25 | 15 | 5 | 110 | 10 | 1.09 | 3 | 28 | 24.6 | 203 | 40 | Toluene | 66.9 | 97 | 99.8 |
| 6 | 30 | 20 | 5 | 115 | 10 | 1.1 | 6 | 30 | 24.6 | 405 | 40 | Toluene | 117.4 | 95 | 99.7 |
| 7 | 35 | 20 | 6 | 120 | 8 | 1.1 | 5 | 30 | 92 | 1270 | 152 | Toluene | 378.5 | 96 | 99.6 |
| 8 | 40 | 25 | 6 | 120 | 8 | 1.11 | 7 | 32 | 92 | 1780 | 160 | Xylene | 508 | 97 | 99.6 |
| 9 | 45 | 30 | 7 | 125 | 6 | 1.12 | 4 | 34 | 220 | 2400 | 380 | Xylene | 750 | 97 | 99.5 |
| 10 | 50 | 30 | 7 | 130 | 6 | 1.13 | 3 | 38 | 220 | 1800 | 380 | Xylene | 600 | 95 | 99.6 |
| 11 | 55 | 35 | 2 | 130 | 15 | 1.13 | 3 | 40 | 518 | 4200 | 880 | Xylene | 1399.5 | 95 | 99.7 |
| 12 | 60 | 40 | 2 | 135 | 15 | 1.14 | 6 | 22 | 518 | 8500 | 880 | Toluene | 2474.5 | 97 | 99.7 |
| 13 | 65 | 40 | 4 | 140 | 25 | 1.16 | 7 | 22 | 518 | 9900 | 900 | Toluene | 2829.5 | 97 | 99.8 |
| 14 | 70 | 45 | 4 | 140 | 25 | 1.18 | 6 | 24 | 1030 | 17000 | 1900 | Xylene | 4982.5 | 98 | 99.5 |
| 15 | 75 | 50 | 4 | 145 | 5 | 1.2 | 7 | 25 | 1030 | 20000 | 1900 | Xylene | 5732.5 | 96 | 99.5 |
| 16 | 80 | 50 | 6 | 150 | 5 | 1.22 | 3 | 25 | 1030 | 9000 | 1900 | Xylene | 2982.5 | 97 | 99.6 |
| 17 | 85 | 55 | 6 | 150 | 5 | 1.24 | 5 | 22 | 3000 | 41000 | 5600 | Toluene | 12400 | 96 | 99.5 |
| 18 | 90 | 60 | 3 | 155 | 4 | 1.26 | 5 | 22 | 3000 | 41000 | 5800 | Toluene | 12450 | 95 | 99.7 |
| 19 | 95 | 60 | 3 | 160 | 4 | 1.28 | 7 | 28 | 3000 | 58000 | 5800 | Xylene | 16700 | 95 | 99.7 |
| 20 | 100 | 65 | 5 | 160 | 4 | 1.3 | 7 | 28 | 7100 | 140000 | 14000 | Xylene | 40275 | 96 | 99.6 |

| Embo diment | Salt forming temperatu re | Diazotizat ion temperat ure | Time of step 1 | Hydrolysis temperatu re | Time of step 2 | Sodium nitrite equivalent s | Sulfuri c acid equival ents | Sulfuric acid solution concentr ation | Aniline compound * | Sulphuric acid solution^{∗} | Sodium nitrite solution^{∗} | Organic solvent | Organic solvent | Yield of produ ct | Purity of produ ct |
|---|---|---|---|---|---|---|---|---|---|---|---|---|---|---|---|
| | °C | °C | s | °C | min | eq. | eq. | % | ml/min | ml/min | ml/min | | **ml/min** | % | % |
| 21 | 105 | 70 | 5 | 165 | 3 | 1.32 | 6 | 30 | 12600 | 200000 | 25000 | Xylene | 59400 | 97 | 99.8 |
| 22 | 110 | 70 | 4 | 170 | 3 | 1.34 | 4 | 30 | 12600 | 140000 | 25000 | Toluene | 44400 | 96 | 99.8 |
| 23 | 115 | 75 | 4 | 170 | 3 | 1.36 | 4 | 32 | 22400 | 250000 | 47000 | Toluene | 79850 | 96 | 99.6 |
| 24 | 120 | 80 | 6 | 175 | 3 | 1.38 | 5 | 32 | 3000 | 41000 | 5600 | Toluene | 2982.5 | 97 | 99.6 |
| 25 | 125 | 80 | 6 | 180 | 2 | 1.4 | 3 | 38 | 22400 | 180000 | 47000 | Toluene | 2982.5 | 97 | 99.7 |
| 26 | 130 | 85 | 7 | 180 | 2 | 1.42 | 3 | 21 | 1030 | 9000 | 1900 | Toluene | 114500 | 96 | 99.7 |
| 27 | 135 | 90 | 7 | 185 | 1 | 1.44 | 4 | 21 | 12600 | 140000 | 25000 | Xylene | 44400 | 95 | 99.6 |
| 28 | 140 | 90 | 5 | 190 | 1 | 1.46 | 5 | 24 | 3000 | 41000 | 5800 | Xylene | 12450 | 96 | 99.7 |
| 29 | 145 | 95 | 5 | 195 | 1 | 1.48 | 5 | 24 | 3000 | 41000 | 5800 | Toluene | 12450 | 97 | 99.6 |
| 30 | 150 | 100 | 5 | 200 | 1 | 1.5 | 7 | 25 | 3000 | 41000 | 5600 | Xylene | 12400 | 97 | 99.7 |
| 31 | 5 | 0 | 4 | 101 | 30 | 1.04 | 3 | 25 | 5.17 | 42 | 8 | / | 13.7925 | 85 | 92.4 |
| 32 | 10 | 0 | 4 | 80 | 40 | 1.05 | 3 | 27 | 6.9 | 58 | 10 | Toluene | 18.725 | 83 | 91.7 |
| 33 | 35 | 20 | 3 | 120 | 35 | 1.1 | 5 | 27 | 92 | 1270 | 152 | / | 378.5 | 84 | 92.6 |
| 34 | 40 | 25 | 3 | 90 | 40 | 1.11 | 7 | 22 | 92 | 1780 | 160 | Xylene | 508 | 88 | 93.5 |
| 35 | 85 | 50 | 2 | 70 | 40 | 1.22 | 5 | 22 | 3000 | 41000 | 5600 | / | 12400 | 78 | 90.2 |
| 36 | 105 | 70 | 2 | 60 | 50 | 1.32 | 6 | 22 | 12600 | 200000 | 25000 | Xylene | 59400 | 83 | 93 |
| 37 | 135 | 90 | 2 | 185 | 8 | 1.44 | 4 | 22 | 12600 | 200000 | 25000 | / | 59400 | 87 | 92.4 |
| ^{∗}The feedstock flow rate used in the actual synthesis will deviate from the flow rate listed in the table by ±5%. | | | | | | | | | | | | | | | |

## Claims

1. A preparation method of *m*-trifluoromethylphenol, **characterized by** comprising the following steps:
step (1): in a continuous flow reactor, carrying out a diazotization reaction of sulfate aqueous solution and sodium nitrite aqueous solution to obtain diazonium salt solution; said sulfate aqueous solution is a mixture of sulfuric acid aqueous solution and *m*-trifluoromethylaniline;
in the sulfate aqueous solution, the molar ratio of sulfuric acid in the sulfuric acid aqueous solution and the *m*-trifluoromethylaniline is (3-7): 1;
the molar ratio of sodium nitrite in the sodium nitrite aqueous solution to the m-trifluoromethylaniline is (1.00-1.50): 1;
step (2): in a continuous flow reactor, carrying out a hydrolysis reaction of the diazonium salt solution obtained in the step (1) in the presence of an aromatic hydrocarbon solvent to obtain the *m*-trifluoromethylphenol; the temperature of the hydrolysis reaction is 101°C-200°C;

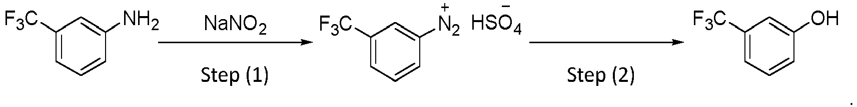

2. The preparation method according to claim 1, **characterized in that** the sulfate aqueous solution is a homogeneous system formed by the sulfuric acid aqueous solution and the *m*-trifluoromethylaniline;
and/or, in the sulfate aqueous solution, the molar ratio of the sulfuric acid and the *m*-trifluoromethylaniline is (3-6): 1;
and/or, the mass percentage concentration of the sulfuric acid aqueous solution is 20%-40%;
and/or, the mass percentage concentration of the sodium nitrite aqueous solution is 20%-35%;
and/or, the molar ratio of the sodium nitrite to the *m*-trifluoromethylaniline in the continuous flow reactor is (1.04-1.15): 1;
and/or, the temperature of the diazotization reaction is 0°C-100°C;
and/or, the residence time of the sulfate aqueous solution and the sodium nitrite aqueous solution in the continuous flow reactor is 5.3-7.5 s;
and/or, the flow rate ratio of the sulfate aqueous solution to the sodium nitrite aqueous solution is 4:1 to 12:1.

3. The preparation method according to claim 2, **characterized in that** the molar ratio of the sulfuric acid to the *m*-trifluoromethylaniline in the sulfate aqueous solution is (3-4):1;
and/or, the mass percentage concentration of the sulfuric acid aqueous solution is 21%-25%;
and/or, the mass percentage concentration of the sodium nitrite aqueous solution is 25%-30%;
and/or, the molar ratio of the sodium nitrite to the *m*-trifluoromethylaniline in the continuous flow reactor is (1.04-1.10):1;
and/or, the temperature of the diazotization reaction is 50°C-70°C;

4. The preparation method according to claim 1, **characterized in that** the aromatic hydrocarbon solvent is toluene and/or xylene;
and/or, the temperature of the hydrolysis reaction is 120°C-150°C;
and/or, the volume ratio of the diazonium salt solution to the aromatic hydrocarbon solvent is (0.1-100): 1;
and/or, the residence time of the hydrolysis reaction is 1 min to 50 min;
and/or, the aromatic hydrocarbon solvent is preheated in a heat exchanger and then mixed with the diazonium salt solution, and then the hydrolysis reaction is carried out in a continuous flow;
and/or, the preparation method further comprises the following post-treatment steps: after the hydrolysis reaction is completely, the reaction system is subjected to oil-water separation to obtain the *m*-trifluoromethyl phenol.

5. The preparation method according to claim 4, **characterized in that** the aromatic hydrocarbon solvent is toluene and/or mixed xylene;
and/or, the volume ratio of the diazonium salt solution to the aromatic hydrocarbon solvent is 4:1 to 20:1;
and/or, the residence time of the hydrolysis reaction is 1 min - 5 min;
and/or, the diazonium salt solution and the aromatic hydrocarbon solvent are mixed and preheated to 90°C-130°C, and then flow into the continuous flow reactor to carry out the hydrolysis reaction;
and/or, when the aromatic hydrocarbon solvent is preheated in a heat exchanger and then mixed with the diazonium salt solution, the residence time of mixing and preheating is 0.5-300 s;
and/or, when the aromatic solvent is preheated in a heat exchanger and then mixed with the diazonium salt solution, the mixing and preheating temperature is 90°C-130°C;
and/or, the preparation method further comprises the following post-treatment steps: after the hydrolysis reaction is completely, the reaction system is subjected to oil-water separation in an oil-water separator to obtain the *m*-trifluoromethyl phenol.

6. The preparation method according to claim 1, **characterized in that**, in the preparation method, the step (1) further comprises the following steps: in a continuous flow reactor, carrying out a salt-forming reaction of the sulfuric acid aqueous solution and the *m*-trifluoromethylaniline to obtain the sulfate aqueous solution;
and/or, the preparation method can further comprise the following post-treatment steps: after the hydrolysis reaction is completely, the reaction system is subjected to oil-water separation, and the oil phase is desolventized and distilled to obtain the m-trifluoromethyl phenol.

7. The preparation method according to claim 6, **characterized in that** the temperature of the salt-forming reaction is 5°C-150°C;
and/or, in the salt-forming reaction, the flow rate ratio of the sulfuric acid aqueous solution to the *m*-trifluoromethylaniline can be 8:1 to 20:1;
and/or, the sulfuric acid aqueous solution and the *m*-trifluoromethylaniline are mixed and preheated in a static mixer separately, and then the salt-forming reaction is carried out.

8. The preparation method according to claim 7, **characterized in that** the temperature of the salt-forming reaction is 85°C to 95°C;
and/or, when the sulfuric acid aqueous solution and the *m*-trifluoromethylaniline are mixed and preheated in a static mixer, the preheating temperature is 85°C to 95°C.

9. The preparation method according to any one of claims 1-8, **characterized in that** the continuous flow reactor is independently a static mixer, a microchannel reactor or a tubular reactor or a combination thereof;
and/or, according to the production scale, one or more groups of the continuous flow reactors are connected in parallel to form a continuous flow reaction system, to prepare the *m*-trifluoromethylphenol.

10. The preparation method according to claim 9, **characterized in that** in the step (1), the continuous flow reactor is a microchannel reactor;
and/or, when the diazonium salt solution and the aromatic hydrocarbon solvent are mixed and preheated in a continuous flow reactor, the continuous flow reactor is a microchannel reactor;
and/or, in the step (2), the continuous flow reactor is a tubular reactor.
